Europäisches Patentamt

European Patent Office

Office européen des brevets

(11) Veröffentlichungsnummer: **0 062 916**
**B1**

(12) **EUROPÄISCHE PATENTSCHRIFT**

(45) Veröffentlichungstag der Patentschrift:
**21.11.84**

(21) Anmeldenummer: **82103077.2**

(22) Anmeldetag: **09.04.82**

(51) Int. Cl.³: **A 61 M 5/30**

(54) **Nadelloses Injektionsgerät.**

(30) Priorität: **16.04.81 DE 3115373**

(43) Veröffentlichungstag der Anmeldung:
**20.10.82 Patentblatt 82/42**

(45) Bekanntmachung des Hinweises auf die Patenterteilung:
**21.11.84 Patentblatt 84/47**

(84) Benannte Vertragsstaaten:
**CH DE FR GB LI**

(56) Entgegenhaltungen:
**Keine Entgegenhaltungen**

(73) Patentinhaber: **HOECHST AKTIENGESELLSCHAFT,
Postfach 80 03 20, D-6230 Frankfurt am Main 80 (DE)**

(72) Erfinder: **Dettbarn, Hans-Jürgen, Rehbocks Ecke 4,
D-3550 Marburg/Lahn (DE)**
Erfinder: **Zimmermann, Josef, Auf der Krautweide 11,
D-6231 Sulzbach (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die Erfindung betrifft ein nadelloses Injektionsgerät mit einer Kolbenpumpe für das zu injizierende Medium und einem Antriebsmotor für die Kolbenpumpe, dessen Arbeitskolben verschiebbar in einer zylindrischen Bohrung des Motorgehäuses angeordnet ist und einen Kolbenschaft aufweist, wobei das Pumpengehäuse mit dem Motorgehäuse und der Pumpenstempel der Kolbenpumpe mit dem Kolbenschaft lösbar verbunden sind.

Injektionsgeräte der genannten Art sind aus der deutschen Auslegeschrift 1 213 958 und der deutschen Offenlegungsschrift 1 922 569 bekannt.

Bei der Injektionsspritze gemäß deutscher Auslegeschrift 1 213 958 wird das Gehäuse der Impfstoffpumpe in der Bohrung des Arbeitskolbens aufgenommen und mittels Verschraubung verbunden. Der Impfstoff-Pumpenkolben weist an seinem Ende ein Gewinde auf und ist damit in den Kolbenschaft des Arbeitskolbens eingeschraubt. Nachteilig ist, daß das Ankuppeln der Impfstoffpumpe in zwei Schritten erfolgt, nämlich zunächst einschrauben des Pumpenkolbens, dann anflanschen des Pumpengehäuses. Dieser Vorgang ist nicht nur zeitraubend, sondern die Impfstoffpumpe verliert auch ihre Sterilität. Beim Abkuppeln der Pumpe vom Motor muß der Pumpenkolben ebenfalls aus dem Pumpengehäuse gezogen werden. Hierbei wird bei aufgesetzter Impfstoffflasche jedoch Impfstoff in die Pumpe gesaugt, der dann beim Abkuppeln verloren geht. Nachteilig für die Funktion der Injektionsspritze ist ferner das Fehlen eines Rammspieles zwischen Pumpe und Motor.

Bei der Impfpistole nach deutscher Offenlegungsschrift 1 922 569 wird die Pumpenkolbenstange mit dem Arbeitskolben durch eine Steckverbindung verbunden. Die Steckverbindung weist ein Kupplungsglied auf, das mit einer sich vom Kupplungsglied auf die Außenseite der Arbeitskolbenstange erstreckenden Betätigungseinrichtung bedient wird. Das Gehäuse der Pumpe wird über Bajonettverschluß mit dem Gehäuse des Arbeitskolbens verbunden. Nachteilig ist, daß beim Kuppeln mehrere Handgriffe gleichzeitig durchgeführt werden müssen. Nachteilig ist ferner, daß die Kolbenstange ungeschützt aus dem Pumpengehäuse heraus ragt und zwar insbesondere mit dem Teil, der während des Schusses mit der Impfstoffkammer in der Kolbenpumpe in Berührung kommt. Sterilität der Impfstoffpumpe ist somit nicht gewährleistet. Nachteilig ist weiterhin das Fehlen eines Rammspieles zwischen Arbeitskolben und Pumpenkolben.

Hier will die Erfindung Abhilfe schaffen. Die Erfindung wie sie in den Ansprüchen gekennzeichnet ist löst die Aufgabe dadurch, daß

a) das Pumpengehäuse am Pumpenkopf eine Ringnut und ein Paar von parallel zueinander liegenden Abflachungen aufweist, die bis zum Grund der Ringnut reichen;

b) der Pumpenstempel am Zapfen, der aus der Pumpenkammer des Pumpengehäuses herausragt, mit zwei gleich tiefen, nebeneinander liegenden Ringnuten und zwei Paaren von jeweils parallel zueinander liegenden Abflachungen versehen ist, die bis zum Grund der Nuten reichen, wobei sich ein Paar der Abflachungen nur von Ringnut zu Ringnut erstreckt und senkrecht zum zweiten Paar der Abflachungen abgeordnet ist;

c) im Motorgehäuse und in einer Bohrung im Kolbenschaft des Antriebsmotors jeweils zwei Stifte parallel zueinander angeordnet sind, die in die Ringnut des Pumpengehäuses und die Ringnuten des Pumpenstempels eingreifen;

d) der Pumpenstempel und der Kolbenschaft mit Einrichtungen gegen Verdrehen in ihren Gehäusen versehen sind.

Die Einrichtung gegen Verdrehen des Pumpenstempels und des Kolbenschaftes in ihren Gehäusen sind zweckmäßig so angeordnet, daß alle Stifte untereinander und das am Pumpenkopf befindliche Paar von Abflachungen mit einem Paar von Abflachungen am Pumpenstempel parallel verlaufen.

Die durch die Erfindung erreichten Vorteile sind im wesentlichen darin zu sehen, daß sichergestellt ist, daß zwischen Arbeitskolben und Pumpenstempel ein vom jeweiligen Kolbenhub unabhängiges Rammspiel vorhanden ist. Dieses Rammspiel ist insofern von Bedeutung, daß hieraus ein Rammstoß resultiert, der einen kurzen Druckstoß erzeugt, der jedoch rasch um das 2- bis 3fache abnimmt. Durch den kurzen Druckstoß wird auf dem zu injizierenden Objekt die für das zu injizierende Medium erforderliche Einschußöffnung mit gewünschter Tiefe erzeugt. Ein weiterer Vorteil ist die synchrone Kupplung der Gehäuse und der Kolben in einer Ebene mit ein und demselben Handgriff. Teile der Pumpe, die steril bleiben müssen, können zu keiner Zeit mit kontaminierten Teilen des Motors oder beim Kuppeln mit der Umgebungsluft in Berührung kommen. Versehentliches Einsaugen von Injektionsmittel in die Kolbenpumpe beim Abkuppeln wird vermieden.

Im folgenden wird die Erfindung an Hand von lediglich einen Ausführungsweg darstellenden Zeichnungen näher erläutert. Es zeigt

Fig. 1 einen Teilausschnitt einer Seitenansicht des nadellosen Injektionsgerätes in abgeschossenem Zustand geschnitten; Kolbenpumpe ist um 90° gegenüber dem Motorgehäuse gedreht,

Fig. 2 den Schnitt II-II der Fig. 1,

Fig. 3 einen Teilausschnitt einer Seitenansicht des nadellosen Injektionsgerätes in gespanntem Zustand geschnitten,

Fig. 4 den Schnitt IV-IV der Fig. 3,

Fig. 5 den Schnitt V-V der Fig. 6,

Fig. 6 die Ansicht des Injektionsgerätes von hinten,

Fig. 7 den Zapfen des Pumpenstempels mit Kolbenschaft,

Fig. 8 die 0°, 45° und 90°-Verdrehung beim Ankuppeln des Pumpenstempels an den Arbeitskolben in der Schnittebene VIII-VIII der Fig. 7,

Fig. 9 die 90°-Verdrehmöglichkeit des Pumpenstempels in der Schnittebene IX-IX der Fig. 7.

Das nadellose Injektionsgerät besteht im wesentlichen aus einer Kolbenpumpe (A) für das zu injizierende Medium, der mit einem Antriebsmotor (B) (nur teilweise gezeigt) lösbar verbunden ist. Das Pumpengehäuse (20) der Kolbenpumpen (A) trägt eine Einrichtung (21) zur Aufnahme eines Behälters für das zu injizierende Medium sowie eine Düse (26). Der Arbeitskolben (5) des Antriebsmotors (B) ist in einer zylindrischen Bohrung (6) des Motorgehäuses (1) verschiebbar angeordnet. Er weist einen Kolbenschaft (4) auf, der in einer Buchse (3) geführt wird. Die Buchse (3) ist in der Bohrung (6) angeordnet.

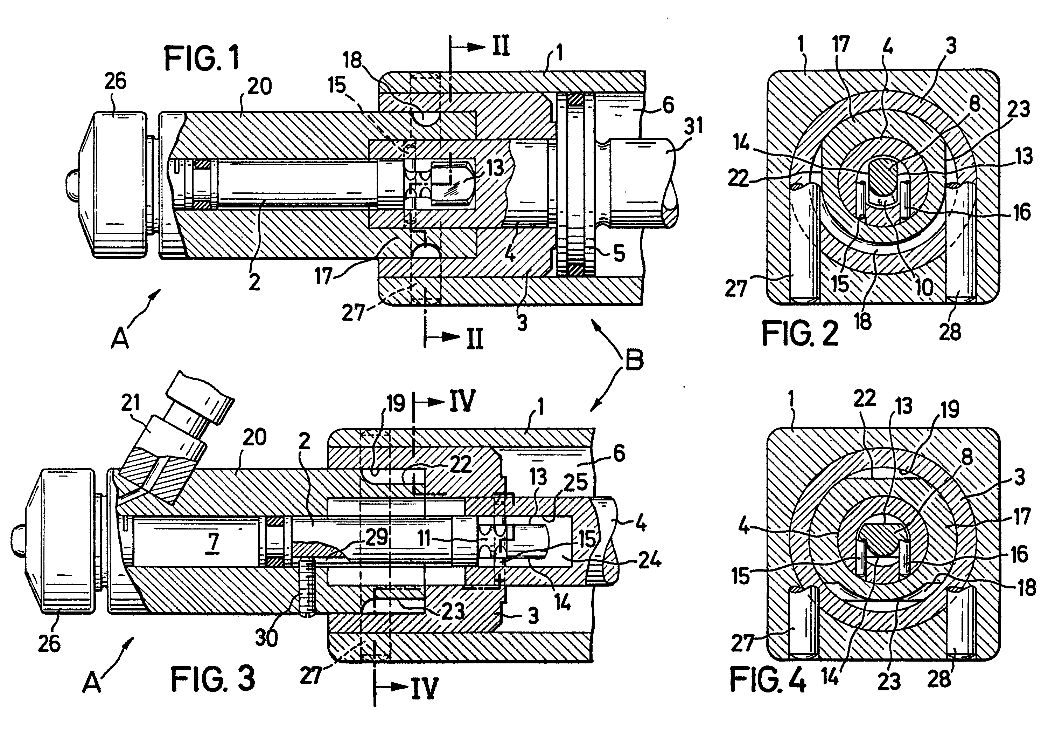

Das Pumpengehäuse (20) der Kolbenpumpe (A) ist mit dem Motorgehäuse (1) des Antriebsmotors (B) und der Pumpenstempel (2) mit dem Kolbenschaft (4) des Arbeitskolbens (5) durch je eine Kupplung verbunden. Die Kupplungen sind so ausgeführt, daß die Kolbenpumpe (A) und der Antriebsmotor (B) durch eine 90°-Drehung in beliebiger Richtung miteinander verbunden oder voneinander getrennt werden können. Beide Teile sind sicher verbunden, wenn man die Kolbenpumpe durch 90°-Drehung im Motorgehäuse (1) einrasten läßt (nicht dargestellt). Die Kupplung zwischen dem Pumpengehäuse (20) und dem Motorgehäuse (1) ist so ausgebildet, daß im Motorgehäuse (1) senkrecht und tangential zur zylindrischen Bohrung (6) Stifte (27) und (28) parallel zueinander angeordnet sind. Am Pumpenkopf (17) des Pumpengehäuses (20) sind eine umlaufende Ringnut (18) sowie zwei Abflachungen (22) und (23), die parallel zueinander liegen und bis zum Grund der Ringnut reichen — d. h. der Abstand der Abflachungen (22) und (23) zueinander ist gleich dem Durchmesser der Ringnut —, so angebracht, daß der Pumpenkopf (17) mit seinen Abflachungen (22) und (23) beim Einschieben in die zylindrische Bohrung (19) der Buchse (3) zwischen die Stifte (27) und (28) paßt und bei einer 90°-Drehung die hintere Flanke der Ringnut (18) hinter die Stifte (27) und (28) zu liegen kommt — d. h. der Abstand der Stifte (27) und (28) ist ebenfalls gleich dem Durchmesser der Ringnut (18) (Fig. 1, 2, 3 und 4).

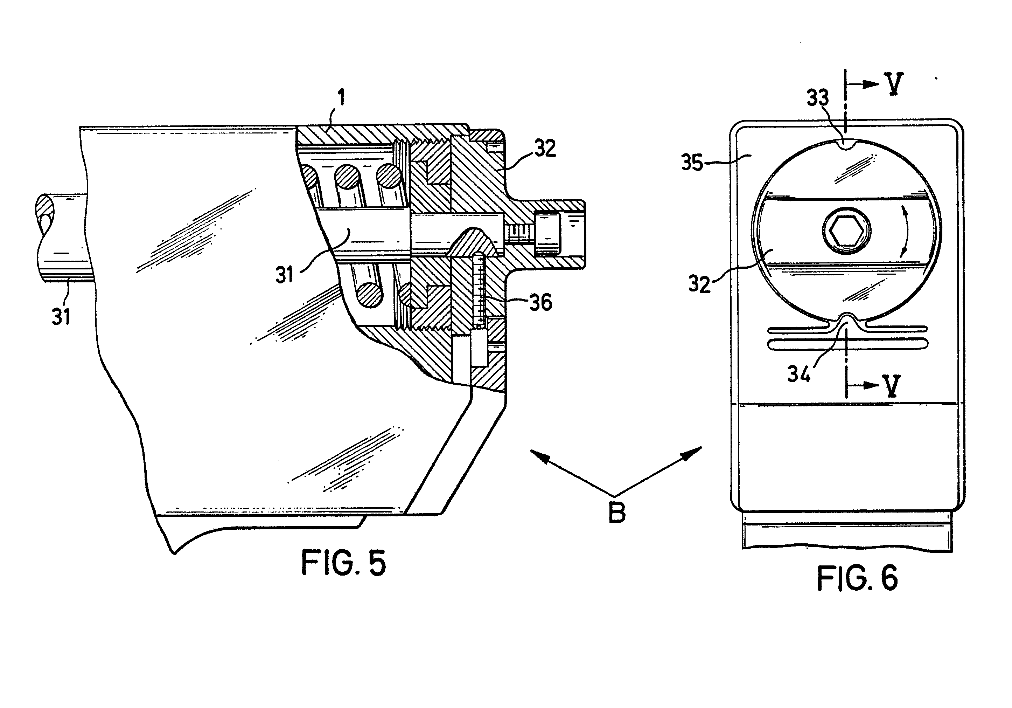

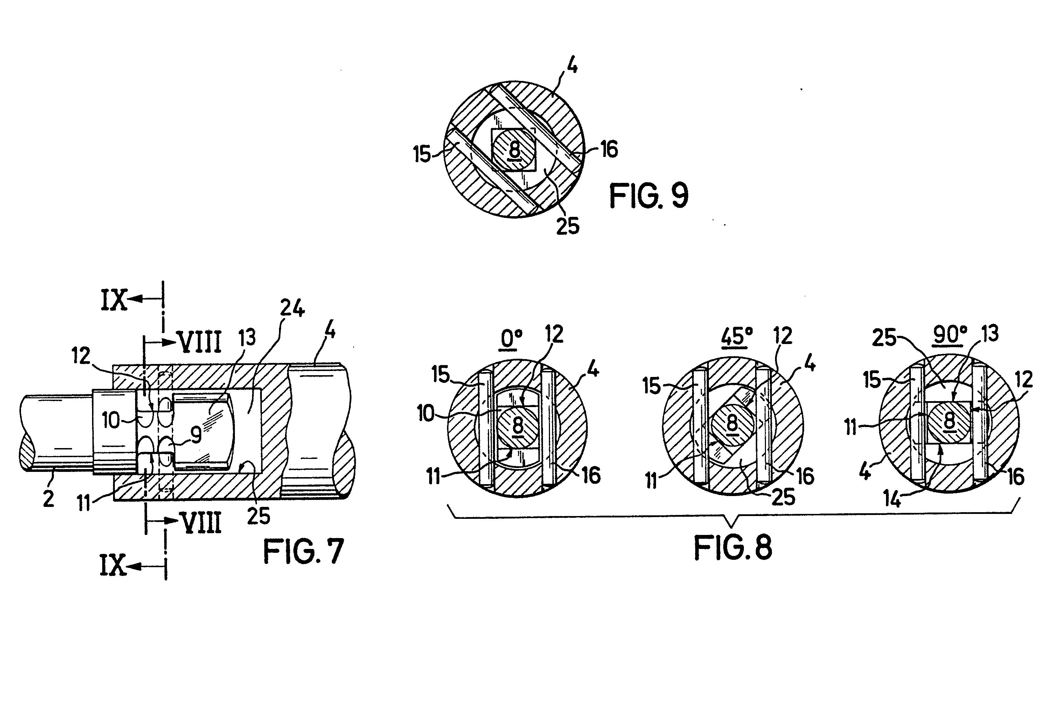

Die Kupplung zwischen Pumpenstempel (2) und dem Kolbenschaft (4) ist im Prinzip ebenso aufgebaut. Der Zpafen (8) des Pumpenstempels (2), der aus der Pumpenkammer (7) des Pumpengehäuses (20) herausragt, ist mit zwei gleich tiefen (gleicher Durchmesser), nebeneinander liegenden Ringnuten (9) und (10) und zwei Paaren von jeweils parallel zueinander liegenden Abflachungen (11, 12, 13 und 14) versehen. Jede Abflachung reicht bis zum Grund der Nuten (9) und (10) — d. h., der Abstand zweier Abflachungen (11) und (12) bzw. (13) und (14) zueinander ist gleich dem Durchmesser der Ringnuten (9) und (10). Die beiden Paare von Anflachungen stehen vorzugsweise senkrecht zueinander. Während die Abflachungen (13) und (14) vom Grund der Ringnut (10) bis zum Ende des Pumpenstempels (2) sich erstrecken, reichen die Abflachungen (11) und (12) lediglich vom Grund der Ringnut (9) zum Grund der Ringnut (10). In einer axialen Bohrung (25) im Kolbenschaft (4) sind senkrecht zur Bohrung zwei Stifte (15) und (16) parallel zueinander angeordnet. Ihr Abstand entspricht etwa dem inneren Durchmesser der Ringnuten (9) und (10) bzw. dem Abstand der Abflachungen (11) und (12) bzw. (13) und (14). Die Fig. 7, 8 und 9 zeigen den in die Bohrung (25) des Kolbenschaftes (4) eingeschobenen Zapfen (8) des Pumpenstempels bei Drehungen des Pumpenstempels (2) um 0°, 45° und 90°. In Fig. 2 ist der Pumpenkopf (17) in die Buchse (3) und der Zapfen (8) des Pumpenstempels (2) in die Bohrung (25) des Kolbenschaftes (4) eingeschoben. Die jeweiligen Abflachungen (22, 23, 11, 12, 13 und 14) stehen parallel zu den zugehörigen Stiften (27, 28, 15 und 16). In Fig. 3 ist die Kolbenpumpe (A) um 90° gegenüber der Stellung gemäß Fig. 1 gedreht und der Arbeitsmotor gespannt. Beim Spannen des Injektionsgerätes gleiten die Stifte (15) und (16) von der Ringnut (10) (Fig. 4 und 7) entlang der Abflachungen (11) und (12) ohne diese zu berühren in die Ringnut (9), an deren Flanke die Stifte hängen bleiben; der Kolbenstempel wird mitgenommen; es entsteht das Rammspiel (24). Dieses Rammspiel ändert sich bei Änderung des Arbeitshubes (Dosiervolumens) der Kolbenpumpe nicht; sein Weg entspricht dem Abstand der beiden Ringnuten (9) und (10). Das Entspannen des Injektionsgerätes und das Abkuppeln der Kolbenpumpe geschieht entsprechend. Dabei ist es zweckmäßig das Ab- und Ankuppeln der Kolbenpumpe (A) an den Antriebsmotor (B) stets bei entspanntem Injektionsgerät vorzunehmen. Für den Kupplungsvorgang ist es erforderlich, daß der Pumpenstempel und der Kolbenschaft gegen Verdrehen in ihren Gehäusen gesichert sind. Solche Einrichtungen zum Sichern können aus einem Schlitz (29) im Pumpenstempel (2) und einem darin gleitenden Sicherungsstift (30) bestehen. Entsprechend kann auch der Pumpenschaft (4) in der Buchse (3) gesichert sein (nicht dargestellt). Eine andere Art der Sicherung gegen Verdrehen des Kolbenschaftes zeigen Fig. 5 und 6. Der Kolbenschaft (4) ist über den Arbeitskolben (5) und die Kolbenstange (31) mit einem Drehknopf (32) verbunden. Der Drehknopf (32) dient zum Einstellen des Hubes für den Arbeitskolben und damit zum Einstellen des Dosiervolumens für das zu injizierende Medium (nicht dargestellt). Der Drehknopf (32) kann an seinem Umfang Nuten (33) aufweisen, in die Feder (34) einrastet, die sich in der Rückwand (35) des Motorgehäuses (1) befindet. Nuten (33) und Feder (34) sind zweckmäßig so aufeinander abgestimmt, daß die Stifte (15) und (16) des Kolbenschaftes parallel zu den Stiften (27) und (28) des Motorgehäuses verlaufen. Selbstverständlich müssen in diesem Fall die Abflachungen (13) und (14) des Pumpenstempels (2) parallel zu den Abflachungen (22) und (23) des Pumpenkopfes ausgerichtet sein. Mit der Schraube (36) wird der Drehknopf (32) mit der Kolbenstange (31) verbunden.

## Patentansprüche

1. Nadelloses Injektionsgerät mit einer Kolbenpumpe (A) für das zu injizierende Medium und einem Antriebsmotor (B) für die Kolbenpumpe (A), dessen Arbeitskolben (5) verschiebbar in einer zylindrischen Bohrung (6) des Motorgehäuses (1) angeordnet ist und einen Kolbenschaft (4) aufweist, wobei das Pumpengehäuse (20) mit dem Motorgehäuse (1) und der Pumpenstempel (2) der Kolbenpumpe (A) mit dem Kolbenschaft (4) lösbar verbunden sind, dadurch gekennzeichnet, daß

a) das Pumpengehäuse (20) am Pumpenkopf (17) eine Ringnut (18) und ein Paar von parallel zueinander liegenden Abflachungen (22, 23) aufweist, die bis zum Grund der Ringnut (18) reichen;

b) der Pumpenstempel (2) am Zapfen (8), der aus der Pumpenkammer (7) des Pumpengehäuses (20) herausragt, mit zwei gleich tiefen, nebeneinander liegenden Ringnuten (9, 10) und zwei Paaren von jeweils parallel zueinander liegenden Abflachungen (11, 12, 13, 14) versehen ist, die bis zum Grund der Ringnuten (9, 10) reichen, wobei sich ein Paar der Abflachungen (11, 12 oder 13, 14) nur von Ringnut (9) zu Ringnut (10) erstreckt und senkrecht zum zweiten Paar der Abflachungen angeordnet ist;

c) im Motorgehäuse (1) und in einer Bohrung (19) im Kolbenschaft (4) jeweils zwei Stifte (15, 16, 27, 28) parallel zueinander angeordnet sind, die in die Ringnut (18) des Pumpengehäuses (20) und die Ringnuten (9) und (10) des Pumpenstempels (2) eingreifen;

d) der Pumpenstempel (2) und der Kolbenschaft (4) mit Einrichtungen (29, 30; 33, 34) gegen Verdrehen in ihren Gehäusen versehen sind.

2. Nadelloses Injektionsgerät nach Anspruch 1, dadurch gekennzeichnet, daß die Einrichtungen (29, 30; 33, 34) gegen Verdrehen des Pumpenstempels (2) und des Kolbenschaftes (4) in ihren Gehäusen so angeordnet sind, daß alle Stifte (15, 16, 27, 28) untereinander und das am Pumpenkopf (17) befindliche Paar von Abflachungen (22, 23) mit einem Paar von Abflachungen (15, 16, 27, 28) am Pumpenstempel (2) parallel verlaufen.

## Claims

1. A needle-less injection instrument with a piston pump (A) for the medium to be injected and with a drive motor (B) for the piston pump (A), the working piston (5) of which is arranged displaceably in a cylindrical bore (6) of the motor housing (1) and has a piston shaft (4), the pump housing (20) being connected releasably to the motor housing (1) and the pump plunger (2) of the piston pump (A) being connected releasably to the piston shaft (4), wherein

a) the pump housing (20) has on the pump head (17) an annular groove (18) and a pair of flattened portions (22, 23) which are parallel to one another and which reach to the bottom of the annular groove (18);

b) the pump plunger (2) on the peg (8) projecting from the pump chamber (7) of the pump housing (20) is provided with two equally deep annular grooves (9, 10) located next to one another and with two pairs of flattened portions (11, 12, 13, 14) which are each parallel to one another and which reach to the bottom of the annular grooves (9, 10), one pair of flattened portions (11, 12 or 13, 14) extending only from the annular groove (9) to the annular groove (10) and being arranged perpendicularly to the second pair of flattened portions;

c) two pins (15, 16, 27, 28) are arranged, parallel to one another, respectively in the motor housing (1) and in a bore (19) in the piston shaft (4) and engage into the annular groove (18) of the pump housing (20) and the annular grooves (9) and (10) of the pump plunger (2); and

d) the pump plunger (2) and the piston shaft (4) are provided with devices (29, 30, 33, 34) preventing them from rotating in their housings.

2. The needle-less injection instrument as claimed in claim 1, wherein the devices (29, 30, 33, 34) preventing the pump plunger (2) and the piston shaft (4) from rotating in their housings are arranged so that all the pins (15, 16, 27, 28) extend parallel to one another and the pair of flattened portions (22, 23) located on the pump head (17) extends parallel to a pair of flattened portions (15, 16, 27, 28) on the pump plunger (2).

## Revendications

1. Appareil d'injection sans aiguille comprenant une pompe à piston (A) pour le milieu à injecter et un moteur d'entraînement (B) pour la pompe à piston (A), dont le piston travaillant (5) est monté coulissant dans un alésage cylindrique (16) du corps (1) du moteur et présente une queue de piston (4), le corps (20) de la pompe étant assemblé au corps (1) du moteur et le plongeur de pompe (2) de la pompe à piston (A) étant assemblé à la queue (4) du piston par des liaisons démontables, caractérisé en ce que

a) le corps de pompe (20) présente au niveau de la tête (17) de la pompe une gorge annulaire (18) et une paire de méplats (22, 23) qui s'étendent parallèlement entre eux et se prolongent jusqu'au fond de la gorge annulaire (18);

b) le plongeur (2) de la pompe est muni, sur le téton (8) qui émerge de la chambre de pompe (7) du corps de pompe (20), de deux gorges annulaires juxtaposées (9, 10), de même profondeur, et de deux paires de méplats (11, 12, 13, 14) s'étendant parallèlement entre eux, qui se prolongent jusqu'au fond des gorges annulaires (9, 10), cependant qu'une paire des méplats (11, 12 ou 13, 14) ne s'étend que de gorge annulaire (9) à gorge annulaire (10) et est disposée perpendiculairement à l'autre paire de méplats;

c) dans le corps (1) du moteur et dans un alésage (19) ménagé dans la queue de piston (4) du moteur travaillant, sont disposées, respectivement parallèlement entre elles, deux goupilles (15, 16, 27, 28) qui s'engagent dans la gorge annulaire (18) du corps de pompe (20) et dans les gorges annulaires (9, 10) du plongeur (2) de la pompe;

d) le plongeur (2) de la pompe et la queue de piston (4) sont munis de dispositifs (29, 30; 33, 34) servant à les bloquer en rotation dans leurs corps.

2. Appareil d'injection sans aiguille selon la revendication 1, caractérisé en ce que les dispositifs (29, 30; 33, 34) servant au blocage du plongeur de pompe (2) et de la queue de piston (4) en rotation dans leurs corps sont agencés de telle manière que toutes les goupilles (15, 16, 27, 28) s'étendant parallèlement entre elles et que la paire de méplats (22, 23) qui se trouvent sur la tête de pompe (17) s'étendent parallèlement à une paire de méplats (15, 16, 27, 28) présents sur le plongeur (2) de la pompe.

FIG. 1

FIG. 2

FIG. 3

FIG. 4

0 062 916

FIG. 5

FIG. 6

FIG. 9

FIG. 7

FIG. 8

0°    45°    90°

0 062 916